(19) Europäisches Patentamt
European Patent Office
Office européen des brevets

(11) Veröffentlichungsnummer: **0 163 965**
**B1**

(12) # EUROPÄISCHE PATENTSCHRIFT

(45) Veröffentlichungstag der Patentschrift: **08.11.89**

(51) Int. Cl.⁴: **C 07 D 237/14, A 61 K 31/50**

(21) Anmeldenummer: **85105535.0**

(22) Anmeldetag: **07.05.85**

(54) **Pyridazinone, ihre Herstellung und Verwendung, Pyridazinone enthaltende Arzeimittel.**

(30) Priorität: **10.05.84 CH 2297/84**

(43) Veröffentlichungstag der Anmeldung:
**11.12.85 Patentblatt 85/50**

(45) Bekanntmachung des Hinweises auf die Patenterteilung:
**08.11.89 Patentblatt 89/45**

(84) Benannte Vertragsstaaten:
**AT BE CH DE FR GB IT LI LU NL SE**

(56) Entgegenhaltungen:
**EP-A- 0 125 636**
**DE-A- 2 445 681**
**DE-A- 2 757 923**
**DE-A- 2 810 267**
**DE-A- 3 417 368**
**US-A- 4 397 854**

**EUROPEAN JOURNAL OF MEDICINAL CHEMISTRY, CHIMICA THERAPEUTICA, Band 9, Nr. 6, November-Dezember 1974, Seiten 644-650, Paris, FR; L. PITARCH et al.: "Etude chimique et pharmacologique d'une série de pyridazones substituées"**
**BULLETIN DE LA SOCIETE CHIMIQUE DE FRANCE, Nr. 2, 1973, Seiten 625-629; J. SCHREIBER et al.: "Cétoacides fonctionnalisés. I. - Amino-alcoylations avec l'acide glyoxylique"**

(73) Patentinhaber: **Byk Gulden Lomberg Chemische Fabrik GmbH, Byk-Gulden-Strasse 2, D-7750 Konstanz (DE)**

(72) Erfinder: **Amschler, Hermann, Dr., Hohenhewenstrasse 19, D-7760 Radolfzell (DE)**
Erfinder: **Eistetter, Klaus, Dr., Säntisblick 7, D-7750 Konstanz 19 (DE)**
Erfinder: **Eltze, Manfrid, Dr., Schützenstrasse 20, D-7750 Konstanz (DE)**
Erfinder: **Flockerzi, Dieter, Dr., Ackerweg 26, D-7753 Allensbach (DE)**
Erfinder: **Kilian, Ulrich, Dr., Stettinerstrasse 22, D-7750 Konstanz (DE)**
Erfinder: **Kolassa, Norbert, Dr., Lerchenweg 12, D-7750 Konstanz 19 (DE)**
Erfinder: **Sanders, Karl, Dr., Felchengang 23, D-7750 Konstanz (DE)**
Erfinder: **Ulrich, Wolf-Rüdiger, Dr., Brandesstrasse 14, D-7750 Konstanz (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Technisches Gebiet

Die Erfindung betrifft Pyridazinone, ihre Herstellung und Verwendung und Pyridazinone enthaltende Arzneimittel.

Stand der Technik

6-Aryl-3[2H]pyridazinone als Ausgangsmaterialien oder Zwischenprodukte für die Synthese von Pharmazeutika und Pflanzenschutzmitteln sowie Verfahren zur ihrer Herstellung werden beispielsweise von Baddar et al. [J. Chem. Soc. 1965, 3342], Steck [J. Heterocycl. Chem. 11 (1974) 755], Albright et al. [J. Heterocycl. Chem. 15 (1978) 881], Schreiber et al. [Bull. Soc. Chim. France 2 (1973) 625]. Pitarch et al. [Eur. J. Med. Chem.-Chimica Therapeutica 9 (1974) 644] und Curran et al. [J. Med. Chem. 17 (1974) 273] beschrieben oder sind u. a. aus folgenden Beschreibungen bekannt: DE-OS 2 435 244, DE-OS 2 445 681, DE-OS 2 757 923.

6-Aryl-3-[2H]pyridazinone mit bestimmter Wirkung sind z, B. aus folgenden Beschreibungen bekannt: DE-OS 2 427 943, DE-OS 2 810 267, DE-OS 2 845 220, EP-OS 8391, EP-OS10 156, JP-OS 58 008 015 und US-PS 4 397 854.

Darstellung der Erfindung

Bestimmte 6-Aryl-3[2H]pyridazinone der allgemeinen Formel I weisen nun eine vorteilhafte pharmakologische Wirkung auf.

Gegenstand der Erfindung sind Verbindungen der allgemeinen Formel I

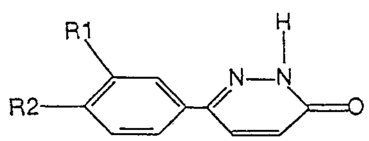

(I),

worin einer der Substituenten R1 und R2 Wasserstoff oder C1–C4-Alkoxy und der andere Polyfluoro-C1–C4 bedeutet,
und ihre pharmakologisch verträglichen Salze mit Basen.

Unter Polyfluor-C1–C4-alkoxy wird eine geradkettige oder verzweigte C1–C4-Alkoxygruppe verstanden, bei der mindestens zwei Wasserstoffatome durch Fluor ersetzt sind. Eine geradkettige C1–C3-Alkoxygruppe, bei der minestens 2 Wasserstoffatome durch Fluor ersetzt sind, ist bevorzugt. Bevorzugte Polyfluoralkoxygruppen sind Difluormethoxy, Trifluormethoxy, 1.1.2.2-Tetrafluorethoxy und 1.1.1-Trifluorethoxy.

C1–C4-Alkoxy ist geradkettig oder verzweigt. Bevorzugt ist die Methoxygruppe.

Als Salze kommen Salze mit anorganischen Basen in Betracht. Als Kationen für die Salzbildung werden vor allem die Kationen der Alkalimetalle oder Erdalkalimetalle verwendet.

Ein weiterer Gegenstand der Erfindung ist die Verwendung der Verbindungen gemäß Anspruch 1 zur Herstellung von Arzneimitteln zur Behandlung oder Prophylaxe von Krankheiten, die auf Erkrankungen der Bronchien und/oder einer Insuffizienz des Herzens beruhen, oder zur Stärkung des Herzens.

Gegenstand der Erfindung ist ferner ein Verfahren zur Herstellung der 6-Aryl-3[2H]pyridazinone der allgemeinen Formel I und ihrer pharmakologisch verträglichen Salze mit Basen, das dadurch gekennzeichnet ist, daß man

a) ein 6-Aryl-tetrahydropyridazinon der allgemeinen Formel II

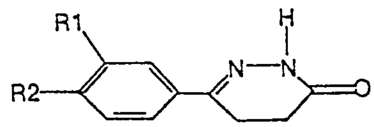

(II),

worin R1 und R2 die oben angegebene Bedeutung haaben, oxydiert und gewünschtenfalls anschließend das erhaltene Pyridazinon in ein Salz überführt, oder

b) eine Morpholinobuttersäure der allgemeinen Formel III

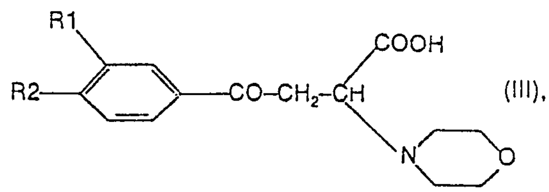

(III),

worin R1 und R2 die oben angegebene Bedeutung haben, mit Hydrazin umsetzt und gewünschtenfalls anschließend das erhaltene Pyridazinon in ein Salz überführt, oder

c) eine Acrylsäure der allgemeinen Formel IV

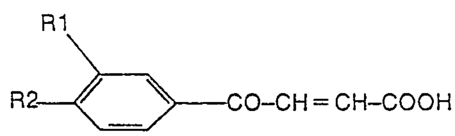

(IV),

worin R1 und R2 die oben angegebene Bedeutung haben, mit Hydrazin umsetzt und gewünschtenfalls anschließend das erhaltene Pyridazinon in ein Salz überführt.

Die Oxydation (Dehydrierung) nach Verfahrensvariante a) erfolgt nach dem Fachmann bekannten Methoden. Beispielsweise kann die Dehydrierung in Gegenwart von Edelmetallen der 8. Nebengruppe, z. B. Palladium oder Platin [DE-OS 2 757 923]; mit Chromtrioxid [Overend et al. J. Chem. Soc. 1947, 239] mit Nitrobenzolsulfonsäuren oder Nitronaphthalinsulfonsäuren, bevorzugt mit deren Natrium- oder Ammoniumsalzen [GB-PS 1 168 291] vorgenommen werden.

Die Umsetzung nach Verfahrensvariante b) erfolgt analog Schreiber et al. [Bull. Soc. chim. France 2(1973)625]. Beispielsweise wird die intermediär gebildete Morpholinobuttersäure III in einem Niederalkanol, z. B. n-Butanol, mit Hydrazinhydrat unter Rückfluß umgesetzt. Alternativ kann das

durch Reaktion des entsprechenden Acetophenons mit Glyoxylsäure und Morpholin erhaltene Morpholiniumsalz der Verbindung III in saurer Lösung mit Hydrazinhydrat umgesetzt werden.

Die Umsetzung nach Verfahrensvariante c) erfolgt nach dem Fachmann bekannten Methoden. Beispielsweise werden die Verbindungen IV analog der DE-OS 2 445 681 in Gegenwart von basischen Verbindungen, wie Alkali metallcarbonaten oder -hydroxiden oder -niederalkanolaten oder tert. Aminen oder Ammoniumhydroxid, mit Methanol oder wäßrigem Methanol bei Raumtemperatur oder leicht erhöhter Temperatur umgesetzt, aus dem entstehenden Salz die Säure in Freiheit gesetzt und diese mit 1 bis 1,5 Mol Hydrazinhydrat erhitzt, wobei mindestens ein neutrales vorzugsweise jedoch saures Milieu aufrechterhalten wird.

Die Überführung der 6-Aryl-3[2H]pyridazinone I in die Salze erfolgt nach dem Fachmann bekannten Methoden. Als alkalischer Reaktionspartner wird diejenige anorganische oder organische Base verwendet, deren Salz gewünscht wird. Man erhält die Salze beispielsweise, indem man die Pyridazinone I mit dem stöchiometrischen Äquivalent an entsprechender Base, z. B. Natriumhydroxid oder Natriummethanolat umsetzt, oder leicht lösliche Salze durch doppelte Umsetzung in schwer lösliche Salze überführt.

Die Verbindungen II, III und IV sind bekannt oder können nach bekannten Verfahren hergestellt werden.

Die folgende Beispiele dienen zur näheren Erläuterung der Erfindung. Fp. bedeutet Festpunkt, Temperaturangaben erfolgen in °C. Die Ausbeuten werden in Prozent der theoretisch berechneten Menge (% d. Th.) angegeben.

Beispiele

Beispiel 1

6-(4-Trifluormethoxyphenyl)-3[2H]pyridazinon

a) 27 g 4-Trifluormethoxyacetophenon werden mit 13,7 g Glyoxylsäuremonohydrat 2 Stunden auf 110° erhitzt. Die Schmelze wird auf 50° abgekühlt, in 100 ml Wasser und 14 ml wäßriger 25%-iger Ammoniaklösung aufgelöst, mit 7 ml Hydrazinhydrat versetzt und 1 Stunde am Rückfluß gekocht. Beim Abkühlen kristallisiert die Titelverbindung aus. Sie wird abgesaugt, im Vakuum getrocknet und aus Isopropanol umkristallisiert. Man erhält 15,9 g (62,1% d. Th.) mit einem Fp. von 200°.

Analog werden hergestellt:

6-[4-(1.1.2.2-Tetrafluorethox)phenyl]-3[2H]pyridazinon, Fp. 178° (80,4% d. Th.),

6-[3-(1.1.2.2-Tetrafluorethoxy)phenyl]-3[2H]pyridazinon, Fp. 155° (70,7% d. Th.)

b) 4-Trifluormethoxyacetophenon

40 g 4-Trifluormethoxybenzoesäure werden in 120 ml Thionylchlorid 3 Stunden am Rückfluß gekocht. Man destilliert überschüssiges Thionylchlorid bei Normaldruck ab und rektifiziert das 4-Trifluormethoxybenzoylchlorid im Vakuum. Man erhält 37,9 g (87% d. Th.) vom Sdp. 82° bei 3,3 kPa.

Aus 4,1 g Magnesiumspänen in 25 ml absolutem Ethanol und 19,9 ml Malonsäuredimethylester und 1 ml Tetrachlorkohlenstoff wird eine Lösung von Magnesiummalonsäuredimethylester bereitet. Zu dieser Lösung werden 37,9 g 4-Trifluormethoxybenzoylchlorid in 85 ml absolutem Ether unter Eiskühlung zugetropft, dann das Kühlbad entfernt und das Reaktionsgemisch über Nacht bei 20° gerührt. Dann werden unter Kühlung mit Eis 25 ml 25%-ige Schwefelsäure zugetropft, die Etherschicht abgetrennt und die wäßrige Phase noch zweimal mit Ether extrahiert. Die vereinigten Etherextrakte werden mit Wasser neutral gewaschen, über Natriumsulfat getrocknet und eingedampft. Das zurückbleibende, gelbe Öl wird in 300 ml 6 N Salzsäure 12 Stunden am Rückfluß gekocht. Nach dem Abkühlen wird das Öl abgetrennt, die wäßrige Phase 6 mal mit je 100 ml Dichlormethan extrahiert, das Öl und die Dichlormethanextrakte vereinigt, über Natriumsulfat getrocknet und eingedampft. Das zurückbleibende Öl wird bei 1,6 kPa destilliert. Man erhält 26,85 g (77,9% d. Th.) der Titelverbindung vom Sdp. 94–96°.

Beispiel 2

6-(4-Trifluormethoxyphenyl)-3[2H]pyridazinon

8,7 g Glyoxylsäuremonohydrat werden in 50 ml Ethanol bis zur vollständigen Lösung aufgekocht, dann werden 16,5 g Morpholin und 19,5 g 4-Trifluormethoxyacetophenon zugefügt und die Mischung 16 Stunden bei 50° gerührt. Die Reaktionsmischung wird im Vakuum eingedampft, der halbfeste Rückstand in 50 ml n-Butanol gelöst, mit 7,0 g Hydrazinhydrat versetzt und 8 Stunden am Rückfluß gekocht. Anschließend wird das Reaktionsgemisch im Vakuum eingedampft, der Rückstand in 100 ml 2 N Salzsäure aufgekocht, abgesaugt und mit Wasser säurefrei gewaschen. Nach Trocknung und Kristallisation aus Isopropanol erhält man 8,4 g (45,4% d. Th.) der Titelverbindung mit dem Fp. 200°.

Beispiel 3

6-[4-(1.1.2.2-Tetrafluorethoxyphenyl)-3[2H]pyridazinon

11,8 g 4-(1.1.2.2-Tetrafluorethoxy)acetophenon und 4,6 g Glyoxylsäuremonohydrat werden gemischt und 1,5 Stunden auf 120° erhitzt. Die Schmelze wird abgekühlt, in 100 ml Methanol gelöst, mit 7,5 g Kaliumcarbonat versetzt und 1 Stunde auf 50–60° erwärmt. Nach Stehen über Nacht wird das Gemisch mit 9,1 ml konzentrierter Salzsäure angesäuert, mit 2,6 ml Hydrazinhydrat versetzt und 3 Stunden am Rückfluß gekocht. Dann wird mit Salzsäure auf pH 1 angesäuert und im Verlauf von 2 Stunden 75 ml Methanol abdestilliert. Man läßt abkühlen, saugt die Kristallmasse ab, schlämmt sie mehrmals mit Wasser ab, saugt ab und trocknet sie schließlich im Vakuum. Man erhält 8,2 g (56,9% d. Th.) der Titelverbindung mit dem FP. 178°.

Beispiel 4

6-(4-Difluormethoxy-3-methoxyphenyl)-3[2H]pyridazinon

a) 15 g 4-Difluormethoxy-3-methoxyacetophenon werden mit 5,9 g Glyoxylsäure-monohydrat 2 Stunden auf 110° erhitzt. Die Schmelze wird anschließend auf 60° abgekühlt, mit 30 ml Wasser versetzt und durch Zusatz von 10 ml konz. wäßriger Ammoniaklösung aufgelöst. Man fügt 3,2 g Hydrazinhydrat zu und kocht die Mischung 2 Stunden am Rückfluß, wobei die Titelverbindung nach und nach auskristallisiert. Nach dem Abkühlen wird der Niederschlag abgesaugt, gut mit Wasser ausgewaschen, getrocknet und aus Isopropanol umkristallisiert. Man erhält 10,8 g (58,1% d. Th.) der Titelverbindung mit dem Fp. 204°.

Analog werden hergestellt:
6-(3-Difluormethoxy-4-methoxyphenyl-3[2H]pyridazinon, Fp. 176° (42,2% d. Th.),
6-(4-Difluormethoxyphenyl)-3[2H]pyridazinon, Fp. 168° (53,9% d. Th.),
6-(3-Difluormethoxyphenyl)-3[2H]pyridazinon, Fp. 170° (87,5% d. Th.).

b) 4-Difluormethoxy-3-methoxyacetophenon

20,8 g 4-Hydroxy-3-methoxyacetophenon werden in 350 ml Dioxan und 350 ml Wasser durch Zugabe von 30 g Ätznatron gelöst und auf 60° erwärmt. In diese Lösung wird unter ständigem Rühren so lange Chlordifluormethan eingeleitet, bis die Gasaufnahme beendet ist (ca. 4 Stunden). Man kühlt die Lösung ab, saugt den entstandenen Niederschlag ab und wäscht ihn dreimal mit je 40 ml Diethylether. Die Lösung wird mit Wasser auf das doppelte Volumen verdünnt und ebenfalls dreimal mit je 100 ml Diethylether extrahiert. Die vereinigten Etherextrakte werden über Magnesiumsulfat getrocknet. Im Vakuum eingedampft und der Rückstand aus Petroleumbenzin (Sdp. 50–70°) kristallisiert. Man erhält 19 g (70,4% d. Th.) 4-Difluormethoxy-3-methoxyacetophenon vom Fp. 68°.

Analog werden hergestellt:
3-Difluormethoxy-4-methoxyacetophenon, Fp. 74° (77,8% d. Th.),
4-Difluormethoxyacetophenon, Öl (78,4% d. Th.),
3-Difluormethoxyacetophenon, Öl (85,7% d. Th.).

Gewerbliche Anwendbarkeit

Die 6-Aryl-3[2H]pyridazinone der allgemeinen Formel I besitzen wertvolle Eigenschaften, die sie gewerblich verwertbar machen. Sie zeichnen sich überraschenderweise durch eine bronchospasmolytische und/oder cardiotonische Wirkung aus, die diejenige des Theophyllin z. T. erheblich übertrifft.

Die bronchospasmolytische Wirksamkeit der 6-Aryl-3[2H]pyridazinone ermöglicht ihren Einsatz in der Human- und Veterinärmedizin, wobei sie zur Behandlung und Prophylaxe von Krankheiten, die auf Erkrankungen der Bronchien beruhen, verwendet werden. Beispielsweise können chronisch obstruktive Atemwegserkrankungen verschiedener Genese (Bronchitis, Asthma bronchiale) bei Mensch und Tier behandelt werden.

Die positiv inotrope Wirksamkeit der 6-Aryl-3[2H]pyridazinone ermöglicht ihren Einsatz in der Human- oder Veterinärmedizin, wobei sie zur Behandlung von Krankheiten, die auf einer Insuffizienz des Herzens beruhen, oder zur Stärkung des Herzens verwendet werden. Beispielsweise werden Herzmuskelschwäche, Herzinsuffizienz, Altersherz, Myokardinfarkt, Herzkreislaufschwäche, Stenokardie bei mangelnder Herzleistung und Koronarinsuffizienz bei Mensch und Tier behandelt.

Ein weiterer Gegenstand der Erfindung ist daher ein Verfahren zur Behandlung von Säugetieren, die an einer der oben genannten Krankheiten erkrankt sind. Das Verfahren ist dadurch gekennzeichnet, daß man dem erkrankten Säugetier eine therapeutisch wirksame und pharmakologisch verträgliche Menge einer oder mehrerer der erfindungsgemäßen Verbindungen verabreicht.

Ein weiterer Gegenstand der Erfindung sind Arzneimittel, die ein oder mehrere der 6-Aryl-3[2H]pyridazinone der allgemeinen Formel I enthalten.

Die Arzneimittel werden nach an sich bekannten Verfahren hergestellt, wobei die Verbindungen als solche oder gegebenenfalls in Kombination mit geeigneten pharmazeutischen Trägerstoffen eingesetzt werden. Enthalten die neuen pharmazeutischen Zubereitungen neben den Wirkstoffen pharmazeutische Trägerstoffe, so beträgt der Wirkstoffgehalt dieser Mischungen 0,5 bis 95, vorzugsweise 15 bis 75 Gewichtsprozent der Gesamtmischung.

Die Wirkstoffe bzw. die Arzneimittel werden in jeder geeigneten Formulierung angewandt unter der Voraussetzung, daß die Ausbildung bzw. Aufrechterhaltung von ausreichenden Wirkstoffspiegeln gewährleistet ist. Das kann beispielsweise durch orale oder parenterale Gabe in geeigneten Dosen erreicht werden. Üblicherweise liegt die pharmazeutische Zubereitung des Wirkstoffs in Form von Einheitsdosen vor, die auf die gewünschte Verabreichung abgestimmt sind. Eine Einheitsdosis kann zum Beispiel eine Tablette, ein Dragee, eine Kapsel, ein Suppositorium oder eine gemessene Volumenmenge eines Pulvers, eines Granulates, einer Lösung, einer Emulsion oder einer Suspension sein.

Unter «Einheitsdosis» im Sinne der vorliegenden Erfindung wird eine physikalisch bestimmte Einheit, die eine individuelle Menge des aktiven Bestandteils in Kombination mit einem pharmazeutischen Trägerstoff enthält, verstanden, deren Wirkstoffgehalt einem Bruchteil oder Vielfachen einer therapeutischen Einzeldosis entspricht. Eine Einzeldosis enthält vorzugsweise die Menge Wirkstoff, die bei einer Applikation verabreicht wird und die gewöhnlich einer ganzen, einer halben, einer drittel oder einer viertel Tagesdosis entspricht. Wenn für eine einzelne therapeutische Verabreichung nur ein Bruchteil, wie die Hälfte oder ein Viertel, der Einheitsdosis benötigt wird, ist die Einheitsdosis vorteilhafterweise teilbar, z. B. in Form einer Tablette mit Bruchkerbe.

Die pharmazeutischen Zubereitungen gemäß der Erfindung können, wenn sie in Einheitsdosen vorliegen und für die Applikation z. B. am Menschen bestimmt sind, etwa 5 bis 250 mg, vorteilhafterweise 10 bis 200 mg und insbesondere 20

bis 100 mg Wirkstoff enthalten. Parenterale Zubereitungen können etwa 1 bis 50 mg, vorteilhafterweise 3 bis 30 mg und insbesondere 5 bis 25 mg Wirkstoff enthalten.

Im allgemeinen werden in der Humanmedizin der oder die Wirksktoffe bei oraler Gabe in einer Tagesdosis von 0,1 bis 10, vorzugsweise 0,3 bis 5, insbesondere 0,5 bis 3 mg/kg Körpergewicht, gegebenenfalls in Form mehrerer, vorzugsweise 1 bis 3 Einzelgaben zur Erzielung der gewünschten Ergebnisse verabreicht. Eine Einzelgabe enthält den oder die Wirkstoffe in Mengen von 0,1 bis 5, vorzugsweise 0,2 bis 3, insbesondere 0,4 bis 2 mg/kg Körpergewicht. Für die inhalative Verabreichung ist es vorteilhaft, den oder die Wirkstoffe in einer Tagesdosis von 0,1 bis 10 mg, vorzugsweise 0,5 bis 5 mg, insbesondere 1 bis 3 mg, gegebenenfalls in Form mehrerer, vorzugsweise 1 bis 3 Einzelgaben zu verabreichen.

Zubereitungen für die intravenöse Verabreichung sind vor allem für die akute Behandlung z. B. Notfallbehandlung, zweckmäßig.

Die therapeutische Verabreichung der pharmazeutischen Zubereitung kann 1 bis 4 mal am Tage zu festgelegten oder variierenden Zeitpunkten erfolgen, z. B. jeweils vor den Mahlzeiten und/oder am Abend. Es kann jedoch erforderlich sein, von den genannten Dosierungen abzuweichen, und zwar in Abhängigkeit von der Art, dem Körpergewicht und dem Alter des zu behandelnden Individuums, der Art und Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Bei akuten Fällen wird zu Beginn der Behandlung eine höhere Dosis verabreicht. Nach Eintreten der gewünschten Wirkung wird auf eine niedrigere Dosis zurückgegangen.

Die Festlegung der jeweils erforderlichen optimalen Dosierung und Applikationsart der Wirkstoffe kann durch jeden Fachmann aufgrund seiner Fachwissens erfolgen.

Die pharmazeutischen Zubereitungen bestehen in der Regel aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z.B. als Vermittler für die Arzneimittelaufnahme durch den Körper, als Formulierungshilfsmittel, als Süßungsmittel, als Geschmackskorrigens, als Farbstoff oder als Konservierungsmittel dienen.

Zur oralen Anwendung können z.B. Tabletten, Dragees, harte und weiche Kapseln, z.B. aus Gelatine, dispergierbare Pulver, Granulate, wässerige und ölige Suspensionen, Emulsionen, Lösungen oder Sirupe kommen.

Tabletten können inerte Verdünnungsmittel, z.B. Calciumcarbonat, Calciumphosphat, Natriumphosphat oder Lactose; Granulierungs- und Verteilungsmittel, z.B. Maisstärke oder Alginate; Bindemittel, z.B. Stärke, Gelatine oder Akaziengummi; und Gleitmittel, z.B. Aluminium- oder Magnesiumstearat, Talkum oder Silikonöl, enthalten. Sie können zusätzlich mit einem Überzug versehen sein, der auch so beschaffen sein kann, daß er eine verzögerte Auflösung und Resorption des Arzneimittels im Gastrointestinaltrakt bewirkt, so daß z.B. eine bessere Verträglichkeit, Protrahierung oder eine Retardierung erreicht wird. Gelatinekapseln können den Arzneistoff vermischt mit einem festen, z.B. Calciumcarbonat oder Kaolin, oder einem öligen, z.B. Oliven-, Erdnuß- oder Paraffinöl, Verdünnungsmittel enthalten.

Wäßrige Suspensionen, die gegebenenfalls kurzfristig zubereitet werden, können Suspendiermittel, z.B. Natriumcarboxymethylcellulose, Methylcellulose, Hydroxypropylcellulose, Natriumalginat, Polyvinylpyrrolidon, Traganthgummi oder Akaziengummi; Dispergier- und Benetzungsmittel, z.B. Polyoxyethylenstearat, Heptadecaethylenoxycetanol, Polyoxyethylensorbitolmonooleat, Polyoxyethylensorbitanmonooleat oder Lecithin; Konservierungsmittel, z.B. Methyl- oder Propylhydroxybenzoate; Geschmacksmittel; Süßungsmittel, z.B. Saccharose, Lactose, Natriumcyclamat, Dextrose, Invertzuckersirup, enthalten.

Ölige Suspensionen können z.B. Erdnuß-, Oliven-, Sesam-, Kokos- oder Paraffinöl und Verdikkungsmittel, wie z.B. Bienenwachs, Hartparrafin oder Cetylalkohol, enthalten; ferner Süßungsmittel, Geschmacksmittel und Antioxidantien.

In Wasser dispergierbare Pulver und Granulate können die Arzneistoffe in Mischungen mit Dispergier-, Benetzungs- und Suspendiermitteln, z.B. den oben genannten, sowie mit Süßungsmitteln, Geschmacksmitteln und Farbstoffen enthalten.

Emulsionen können z.B. Oliven-, Erdnuß- oder Paraffinöl neben Emulgiermitteln, wie z.B. Akaziengummi, Traganthgummi, Phosphatiden, Sorbitanmonooleat, Polyoxyethylensorbitanmonooleat, und Süßungs- und Geschmacksmitteln enthalten.

Zur rektalen Anwendung der Arzneistoffe werden Suppositorien verwendet, die mit Hilfe von bei Rektaltemperatur schmelzenden Bindemitteln, beispielsweise Kakaobutter oder Polyethylenglykol, hergestellt werden.

Zur parenteralen Anwendung der Arzneistoffe dienen steril injizierbare, gegebenenfalls kurzfristig herzustellende, wäßrige Suspensionen, isotonische Salzlösungen oder sonstige Lösungen, die Dispergier- oder Benetzungsmittel und/oder pharmakologisch verträgliche Verdünnungsmittel, z.B. Propylen- oder Butylenglykol, enthalten.

Die orale Anwendung der Arzneistoffe ist bevorzugt.

Bevorzugt für die Verwendung als Bronchospasmolytikum ist auch die inhalative Applikation

der erfindungsgemäßen Verbindungen. Diese werden entweder direkt als Pulver oder durch Vernebeln von die erfindungsgemäßen Verbindungen enthaltenden Lösungen oder Suspensionen verabreicht. Die Vernebelung kann dabei auf herkömmliche Weise, beispielsweise durch Preßluftvernebler oder Ultraschallvernebler, erfolgen. Besonders vorteilhaft ist die Verabreichung aus Sprühdosen, insbesondere solcher mit einem herkömmlichen Dosierventil (Dosier-Aerosole). Mittels Dosier-Aerosolen ist es möglich, pro Sprühstoß eine definierte Menge an Wirkstoff bereitzustellen. Von besonderem Vorteil sind hier sogenannte Synchron-Inhalatoren, womit die Wirkstoffgabe synchron zur Einatmung geschehen kann. Geeignete Synchron-Inhalationsvorrichtungen sind z.B. in der DE-PS 1 945 257, DE-PS 1 917 911 und DE-OS 2 055 734 offenbart.

Für Inhalationszwecke kommen die Wirkstoffe vorzugsweise in mikronisierter Form zum Einsatz, wobei Teilchengrößen von weniger als 10 µm vorteilhaft sind. Zur Applikation aus Sprühdosen werden die Wirkstoffe in üblichen Treibmitteln dispergiert, vorzugsweise unter Zuhilfenahme eines Dispergiermittels. Als Treibmittel kommen insbesondere Gemische von Trichlorfluormethan (Frigen®12) in Frage, wobei Trichlorfluormethan ganz oder teilweise durch 1,1,2-Trichlortrifluorethan (Frigen®113) ersetzt werden kann. Als Dispergiermittel kommen insbesondere die für diese Zwecke gebräuchlichen Sorbitanester (Spane® der Firma Atlas GmbH) und Lecithin in Frage. Das Dispergiermittel wird in der gekühlt vorgelegten schwerer flüchtigen Treibmittelkomponente gelöst. In die Lösung wird der mikronisierte Wirkstoff bzw. werden die mikronisierten Wirkstoffe eingerührt. Die Dispersion wird in Sprühbüchsen eingefüllt. Nach dem Vercrimpen wird die leichter flüchtige Treibmittelkomponente aufgedrückt.

Der oder die Wirkstoffe können gegebenenfalls mit einem oder mehreren der angegebenen Träger- oder Zusatzstoffe auch in mikroverkapselter Form formuliert werden.

Tabletten mit 100 mg
6-(4-Difluormethoxy-3-methoxyphenyl)-3[2H]pyridazinon

40 kg Wirkstoff, 24 kg Milchzucker und 16 kg Maisstärke werden mit 4 kg Polyvinylpyrrolidon (MG 25 000) in 5,5 Liter Wasser granuliert und durch ein Sieb von 1,25 mm Maschenweite gepreßt. Nach dem Trocknen werden 10 kg Carboxymethylcellulose, 4 kg Talkum und 2 kg Magnesiumstearat zugegeben. Auf einer Exzentermaschine wird das Granulat zu Tabletten von 9 mm Durchmesser, 250 mg Gewicht und einer Härte von 4 bis 5 kg verpreßt.

Kapseln mit 15 mg
6-(3-Difluormethoxyphenyl)-3[2H]pyridazinon

150 mg Wirkstoff, 845 mg mikrokristalline Cellulose und 5 mg amorphe Kieselsäure werden feingepulvert, gut vermischt und in Hartgelatinekapseln Größe 4 abgefüllt.

Dosieraerosolzubereitung enthaltend 6-[3-(1,1,2,2-Tetrafluorethoxyphenyl)]-3[2H]pyridazinon

0,540 g Span®85 und 0,135 g Aroma werden in 10,215 g gekühltem Frigen®11 gelöst. In die Lösung werden 0,270 g mikronisierter Wirkstoff eingerührt und in 24 ml-Dosen eingefüllt. Nach dem Vercrimpen werden 14,971 g Frigen®12 eingepreßt. Bei einem Kammervolumen des Dosierventils von 125 µl werden pro Ventilhub 1,6 mg Wirkstoff als Aerosol freigesetzt.

Biologische Untersuchungen

Die 6-Aryl-3[2H]pyridazinone der allgemeinen Formel I weisen eine bronchospasmolytische Wirkung auf, welche diejenige des Theophyllins zum Teil erheblich übertrifft. Außerdem weisen sie im Vergleich zu Theophyllin eine höhere organselektive Wirkung auf, wie der Vergleich Bronchospasmolyse an der isolierten, spontan kontrahierten Trachealspangenkette des Meerschweinchens zur positiv inotropen Wirkung am elektrisch gereizten linken Vorhof der Ratte erkennen läßt. Theophyllin wird seit langer Zeit als Broncholytikum bei der Behandlung des Asthma bronchiale und anderen spastischen Zuständen der glatten Bronchialmuskulatur verwendet. Seine Nebenwirkungen, besonders die auf Herz und Kreislauf, sind bekannt [vergl. Ehrhart/Ruschig, Arzneimittel, Verlag Chemie Weinheim/Bergstraße 1972, Bd. 1, Seite 341, Bd. 2, Seite 258; Schulze-Wernighaus Pharmakotherapie 4 (1981) 168 bis 177].

Die Verbindungen werden in den folgenden Tabellen durch eine laufende Nummer bezeichnet.

1: 6-(4-Trifluormethoxyphenyl)-3[2H]pyridazinon
2: 6-[3-(1.1.2.2-Tetrafluorethoxy)phenyl]-3[2H]pyridazinon
3: 6-[4-(1.1.2.2-Tetrafluorethoxy)phenyl]-3[2H]pyridazinon
4: 6-(3-Difluormethoxy-4-methoxyphenyl)-3-[2H]pyridazinon
5: 6-(4-Difluormethoxy-3-methoxyphenyl)-3[2H]pyridazinon
6: 6-(4-Difluormethoxyphenyl)-3[2H]pyridazinon
7: 6-(3-Difluormethoxyphenyl)-3[2H]pyridazinon

Die bronchospasmolytische Wirkung der Verbindungen auf die Trachealspangenkette des Meerschweinchens wurde in vitro geprüft:

Vier parallele, aus jeweils 6 Einzelringen bestehende Trachealspangen-Ketten des Meerschweinchens (♂ und ♀, 430–600 g) im Organbad [5 ml, Krebs-Henseleit-Lösung mit Zusatz von Phentolamin ($10^{-5}$ mol/l), 37°, Vorspannung der Organe 2 g, Begasung mit Carbogen] entwickeln nach etwa 20 bis 30 Minuten eine stabile, tonische Spontankontraktur. An diesen dauerkontrahierten Organen kann unter isometrischen Meßbedingungen durch Applikation der Prüfsubstanz in kumulativ-halblogarithmisch ansteigender Konzentration (z.B. $1 \times 10^{-6} + 2 \times 10^{-6} + 7 \times 10^{-5}$ usw. mol/l) eine Relaxation herbeigeführt werden, wobei nach jeder Einzeldosis der Testsubstanz eine konstante Relaxations-Antwort abgewartet wird, bevor die nächst höhere Konzentration appliziert wird. Über einen Zeitraum von 20 bis 30 Minuten wird somit eine vollständige Dosis-Wirkungskurve

der Testsubstanz erhalten. Die jeweilige Relaxation wird als Prozentbruchteil der durch Gabe von (–)Isoprenalin ($10^{-6}$ mol/l) maximal erreichbaren Relaxation ausgedrückt. Als Maß für die bronchodilatorische Aktivität dient die Konzentration der Testsubstanz, welche 50% der maximal erreichbaren Relaxation bewirkt, ausgedrückt durch den negativen Logarithmus der $EC_{50}$ mol/l: –1 g[$EC_{50}$].

Die positiv inotrope Wirkung der Verbindungen wurde am linken, elektrisch gereizten Vorhof der Ratte in vitro geprüft:

Registriert wurden isometrische Kontraktionen (HSE-Kraftaufnehmer K-30; Watanabe-Schreiber Linear Corder Mark 5) isolierter, linker Vorhöfe von Ratten (♂, 250–300 g) im Organbad (10 ml, Tyrode-Nährlösung, 31°, Begasung mit Carbogen, Vorspannung der Organe 0,25 g) unter elektrischer Stimulation (HSE-Reizgerät, 7 V, 3 ms, 2 Hz). Nach einer Äquilibrierungszeit von 30 Minuten kann durch Applikation der Prüfsubstanz in kumulativ-halblogarithmisch ansteigender Konzentration (z.B. $1 \times 10^{-6} + 2 \times 10^{-6} + 7 \times 10^{-5}$ usw. mol/l) eine dosisabhängige Zunahme der Kontraktionskraft herbeigeführt werden, wobei nach jeder Einzeldosis der Testsubstanz eine konstante Inotropie-Antwort abgewartet wird, bevor die nächsthöhere Konzentration appliziert wird. Die jeweilige Zunahme der Kontraktionskraft wird in % zum Ausgangswert vor der Substanzapplikation ausgedrückt. Als Maß für die kardiotonische Aktivität dient die Konzentration der Testsubstanz, welche die Kontraktionskraft des Vorhofes um 40% über den Ausgangswert verstärkt [$EC_{40pot.}$ mol/l], ausgedrückt durch den negativen Logarithmus der [$EC_{40pot.}$ mol/l]: –1 g[$EC_{40pot.}$].

Als Maß für die organselektive Wirksamkeit dient der Quotient aus $[EC_{40pot.}]_{linker\ Vorhof}$ und $[EC_{50}]_{Trachea}$. Als Maß für die Wirksamkeit werden die Quotienten der $[EC_{50}]_{Trachea}$-Werte bzw. $[EC_{40pot.}]_{linker\ Vorhof}$-Werte von Theophyllin und geprüfter Verbindung angegeben.

Die gefundenen Werte sind in der nachfolgenden Tabelle 1 aufgeführt:

Tabelle 1
Bronchospasmolytische und positiv inotrope Wirkung

| lfd. Nr. | A | B | C | D | E |
|---|---|---|---|---|---|
| 1 | 4,60 | 4,0 | 4,0 | 5,4 | 4,2 |
| 2 | 5,11 | 4,66 | 2,8 | 17,4 | 19 |
| | 5,04 | 4,21 | 6,8 | 13,2 | 6,8 |
| 4 | 5,80 | 5,76 | 1,1 | 85 | 240 |
| 5 | 5,91 | 6,06 | 0,7 | 110 | 469 |
| 6 | 4,92 | 4,49 | 2,7 | 11,2 | 12,9 |
| 7 | 4,84 | <4,0 | >6,9 | 9,3 | - |
| Theophyllin | 3,87 | 3,38 | 3,1 | 1 | 1 |

Spalte A: –lg $[EC_{50}]_{Trachea}$
Spalte B: –lg $[EC_{40pot}]_{linker\ Vorhof}$
Spalte C: $[EC_{40pot.}]_{linker\ Vorhof}/[EC_{50}]_{Trachea}$
Spalte D: $[EC_{50}]_{Theophyllin}/[EC_{50}]_{Substanz}$
Spalte E: $[EC_{40pot.}]_{Theophyllin}/[EC_{40pot.}]_{Substanz}$

Die Schutzwirkung gegen der Acetylcholininduzierten Bronchospasmus wurde am wachen Meerschweinchen in Anlehnung an T. Olsson, Acta Allergologica 26, 438–447 (1971) bestimmt:

Meerschweinchen (250–350 g) werden in einem verschlossenen Plexiglaszylinder (Volumen: 5 l) zweimal im Abstand von 20 Minuten einem Acetylcholin-Nebel (0,06% in 0,9% Natriumchloridlösung; Ultraschallvernebler Heyer Use 77) ausgesetzt. Die Zeit vom Beginn der Vernebelung bis zum Einsetzen deutlicher Atemanstrengungen (unter Umständen hypoxischer Krampfanfall in Seitenlage) wird gemessen und als Latenzzeit bezeichnet.

Im Kontrollversuch (ohne Substanzapplikation) liegt die Latenzzeit bei 2 Minuten. Die Applikation der Prüfsubstanz erfolgt per oral mittels Schlundsonde (Standarddosis 100 µmol/kg, Volumen 1 ml 4%ige Methocelsuspension) in 0,9%iger Natriumchloridlösung/kg. Nach 30 Minuten werden die Tiere erneut dem Acetylcholin-Nebel ausgesetzt und die Latenzzeiten gemessen. Eine Verlängerung der Latenzzeit auf mindestens die zweifache Länge wird als Schutzwirkung angesehen.

Die gefundenen Werte sind in der nachfolgenden Tabelle 2 aufgeführt:

Tabelle 2
Schutzwirkung gegen den Acetylcholin-induzierten Bronchospasmus am wachen Meerschweinchen

| lfd. Nr. | Dosis [µmol/kg]p.o.* | Versuch 30 Min. p.a. Verdreifachung der Latenzzeit bei N von 10 Tieren |
|---|---|---|
| 2 | 100 | 8 |
| | 100 | 7 |
| 5 | 100 | 9 |
| 6 | 100 | 8 |
| 7 | 100 | 9 |
| Theophyllin | 100 | 3 |

*appliziert in 4-proz. Methocel-Suspension

Aus der Tabelle 2 ergibt sich, daß die untersuchten Verbindungen eine höhere Schutzwirkung gegen den durch Acetylcholin-Vernebelung erzeugten Bronchospasmus beim wachen Meerschweinchen bewirken als die Vergleichssubstanz Theophyllin.

**Patentansprüche für die Vertragsstaaten: BE, CH, LI, DE, FR, GB, IT, LU, NL, SE**

1. Verbindungen der allgemeinen Formel I

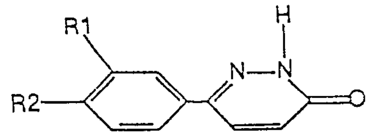

(I),

worin einer der Substituenten R1 und R2 Wasserstoff oder C1–C4-Alkoxy und der andere Polyfluoro-C1–C4-alkoxy bedeutet, und ihre pharmakologisch verträglichen Salze mit Basen.

2. Verbindungen nach Anspruch 1, dadurch gekennzeichnet, daß in der allgemeinen Formel I einer der Substituenten R1 und R2 Wasserstoff oder geradkettiges C1–C3-Alkoxy und der andere Polyfluor-C1–C2-alkoxy bedeutet, und ihre pharmakologisch verträglichen Salze mit Basen.

3. Verbindungen nach Anspruch 1, dadurch gekennzeichnet, daß in der allgemeinen Formel I einer der Substituenten R1 und R2 Wasserstoff oder Methoxy und der andere Difluormethoxy, Trifluormethoxy, 1.1.2.2-Tetrafluorethoxy oder 1.1.1-Trifluorethoxy bedeutet.

4. 6-(4-Difluormethoxy-3-methoxyphenyl)-3[2H]pyridazinon.

5. Verfahren zur Herstellung der Verbindungen nach Anspruch 1, dadurch gekennzeichnet, daß man

a) ein 6-Aryl-tetrahydropyridazinon der allgemeinen Formel II

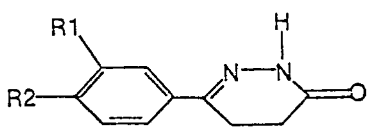 (II),

worin R1 und R2 die oben angegebene Bedeutung haben, oxydiert und gewünschtenfalls anschließend das erhaltene Pyridazinon in ein Salz überführt, oder

b) eine Morpholinbuttersäure der allgemeinen Formel III

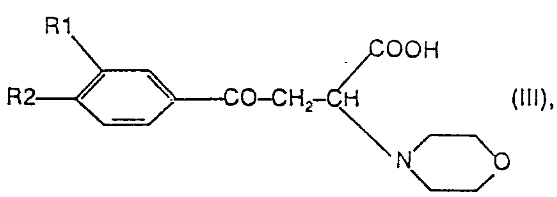 (III),

worin R1 und R2 die oben angegebene Bedeutung haben, mit Hydrazin umsetzt und gewünschtenfalls anschließend das erhaltene Pyridazinon in ein Salz überführt, oder

c) eine Acrylsäure der allgemeinen Formel IV

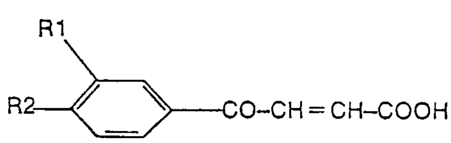 (IV),

worin R1 und R2 die oben angegebene Bedeutung haben, mit Hydrazin umsetzt und gewünschtenfalls anschließend das erhaltene Pyridazinon in ein Salz überführt.

6. Arzneimittel enthaltend eine oder mehrere Verbindungen nach Anspruch 1 und/oder deren pharmakologisch verträglichen Salze.

7. Arzneimittel nach Anspruch 6 zur Behandlung von Erkrankungen der Bronchien.

8. Arzneimittel nach Anspruch 7 enthaltend 6-(4-Difluormethoxy-3-methoxyphenyl)-3[2H]pyridazinon und/oder deren pharmakologisch verträglichen Salze.

9. Verwendung der Verbindungen nach Anspruch 1 und/oder ihrer pharmakologisch verträglichen Salze zur Herstellung von Arzneimitteln zur Behandlung oder Prophylaxe von Krankheiten, die auf Erkrankungen der Bronchien und/oder einer Insuffizienz des Herzens beruhen, oder zur Stärkung des Herzens.

10. Verwendung von 6-(4-Difluormethoxy-3-methoxyphenyl)-3[2H]pyridazinon und/oder dessen pharmakologisch verträglichen Salze zur Herstellung von Arzneimitteln zur Behandlung oder Prophylaxe von Krankheiten, die auf Erkrankungen der Bronchien und/oder einer Insuffizienz des Herzens beruhen, oder zur Stärkung des Herzens.

**Patentansprüche für den Vertragsstaat: AT**

1. Verfahren zur Herstellung von Verbindungen der allgemeinen Formel I

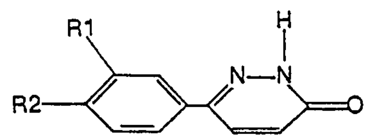 (I),

worin einer der Substituenten R1 und R2 Wasserstoff oder C1–C4-Alkoxy und der andere Polyfluoro-C1–C4-alkoxy bedeutet, und ihren pharmakologisch verträglichen Salzen in Basen, dadurch gekennzeichnet, daß man

a) ein 6-Aryl-tetrahydropyridazinon der allgemeinen Formel II

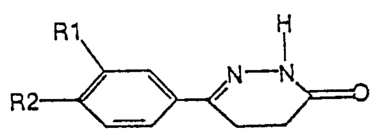 (II),

worin R1 und R2 die oben angegebene Bedeutung haben, oxydiert und gewünschtenfalls anschließend das erhaltene Pyridazinon in ein Salz überführt, oder

b) eine Morpholinbuttersäure der allgemeinen Formel III

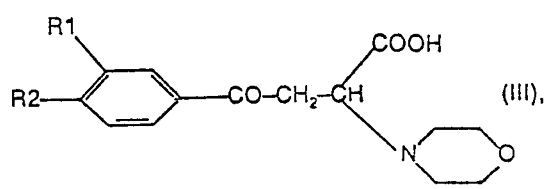 (III),

worin R1 und R2 die oben angegebene Bedeutung haben, mit Hydrazin umsetzt und gewünschtenfalls anschließend das erhaltene Pyridazinon in ein Salz überführt, oder

c) eine Acrylsäure der allgemeinen Formel IV

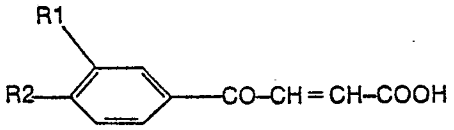

(IV),

worin R1 und R2 die oben angegebene Bedeutung haben, mit Hydrazin umsetzt und gewünschtenfalls anschließend das erhaltene Pyridazinon in ein Salz überführt.

2. Verfahren nach Anspruch 1 zur Herstellung von Verbindungen der allgemeinen Formel I, worin einer der Substituenten R1 und R2 Wasserstoff oder geradkettiges C1–C3-Alkoxy und der andere Polyfluor-C1–C2-alkoxy bedeutet, und ihren pharmakologisch verträglichen Salzen mit Basen, dadurch gekennzeichnet, daß man

a) ein 6-Aryl-tetrahydropyridazinon der allgemeinen Formel II worin R1 und R2 die oben angegebene Bedeutung haben, oxydiert und gewünschtenfalls anschließend das erhaltene Pyridazinon in ein Salz überführt, oder

b) eine Morpholinbuttersäure der allgemeinen Formel III worin R1 und R2 die oben angegebene Bedeutung haben, mit Hydrazin umsetzt und gewünschtenfalls anschließend das erhaltene Pyridazinon in ein Salz überführt, oder

c) eine Acrylsäure der allgemeinen Formel IV worin R1 und R2 die oben angegebene Bedeutung haben, mit Hydrazin umsetzt und gewünschtenfalls anschließend das erhaltene Pyridazinon in ein Salz überführt.

3. Verfahren nach Anspruch 1 zur Herstellung von Verbindungen der allgemeinen Formel I, worin einer der Substituenten R1 und R2 Wasserstoff oder Methoxy und der andere Difluormethoxy, Trifluormethoxy, 1.1.2.2-Tetrafluorethoxy oder 1.1.1-Trifluorethoxy bedeutet, und ihren pharmakologisch verträglichen Salzen mit Basen, dadurch gekennzeichnet, daß man

a) ein 6-Aryl-tetrahydropyridazinon der allgemeinen Formel II worin R1 und R2 die oben angegebene Bedeutung haben, oxydiert und gewünschtenfalls anschließend das erhaltene Pyridazinon in ein Salz überführt, oder

b) eine Morpholinbuttersäure der allgemeinen Formel III worin R1 und R2 die oben angegebene Bedeutung haben, mit Hydrazin umsetzt und gewünschtenfalls anschließend das erhaltene Pyridazinon in ein Salz überführt, oder

c) eine Acrylsäure der allgemeinen Formel IV worin R1 und R2 die oben angegebene Bedeutung haben, mit Hydrazin umsetzt und gewünschtenfalls anschließend das erhaltene Pyridazinon in ein Salz überführt.

4. Verfahren nach Anspruch 1 zur Herstellung von 6-(4-Difluormethoxy-3-methoxyphenyl)-3[2H]pyridazinon, und dessen pharmakologisch verträglichen Salzen mit Basen, dadurch gekennzeichnet, daß man

a) 6-(4-Difluormethoxy-3-methoxyphenyl)-tetrahydropyridazin-3-on oxydiert und gewünschtenfalls anschließend das erhaltene Pyridazinon in ein Salz überführt, oder

b) 4-(4-Difluormethoxy-3-methoxyphenyl)-2-morpholino-4-oxobuttersäure mit Hydrazin umsetzt und anschließend das erhaltene Pyridazinon in ein Salz überführt, oder

c) 2,3-Dehydro-4-(4-difluormethoxy-3-methoxyphenyl)-4-oxobuttersäure mit Hydrazin umsetzt und gewünschtenfalls das erhaltene Pyridazinon anschließend in ein Salz überführt.

5. Verfahren zur Herstellung von Arzneimitteln zur Behandlung oder Prophylaxe von Krankheiten, die auf Erkrankungen der Bronchien und/oder einer Insuffizienz des Herzens beruhen, oder zur Stärkung des Herzens, dadurch gekennzeichnet, daß man ein oder mehrere Verbindungen der in Anspruch 1 angegebenen allgemeinen Formel I und/oder deren pharmakologisch verträglichen Salze mit Basen durch Mischen mit geeigneten pharmazeutisch annehmbaren Trägerstoffen in eine zur Verabreichung als Arzneimittel geeignete Form bringt.

6. Verfahren nach Anspruch 5, dadurch gekennzeichnet, daß man 6-(4-Difluormethoxy-3-methoxyphenyl)-3[2H]pyridazinon und/oder dessen pharmakologisch verträglichen Salze mit Basen verwendet.

7. Verwendung der Verbindungen der in Anspruch 1 angegebenen allgemeinen Formel I und deren pharmakologisch verträglichen Salze mit Basen bei der Herstellung von Arzneimitteln zur Behandlung oder Prophylaxe von Krankheiten, die auf Erkrankungen der Bronchien beruhen.

8. Verwendung von 6-(4-Difluormethoxy-3-methoxyphenyl)-3[2H]pyridazinon und dessen pharmakologisch verträglichen Salze mit Basen bei der Herstellung von Arzneimitteln zur Behandlung oder Prophylaxe von Krankheiten, die auf Erkrankungen der Bronchien beruhen.

**Claims for the contracting states: BE, CH, LI, DE, FR, GB, IT, LU, NL, SE**

1. Compounds of the general formula I

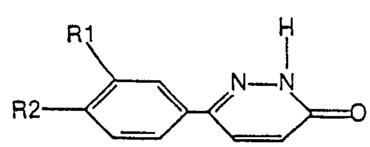

(I)

in which one of the substituents R1 and R2 denotes hydrogen or C1–C4-alkoxy, and the other denotes polyfluoro-C1–C4-alkoxy, and their pharmacologically tolerated salts with bases.

2. Compounds as claimed in claim 1, wherein, in general formula I, one of the substituents R1 and

R2 denotes hydrogen or straight-chain C1–C3-alkoxy, and the other denotes polyfluoro-C1–C2-alkoxy, and their pharmacologically tolerated salts with bases.

3. Compounds as claimed in claim 1, wherein, in general formula I, one of the substituents R1 and R2 denotes hydrogen or methoxy, and the other denotes difluoromethoxy, trifluoromethoxy, 1,1,2,2-tetrafluoroethoxy or 1,1,1-trifluoroethoxy.

4. 6-(4-Difluoromethoxy-3-methoxyphenyl)-3[2H]pyridazinone.

5. A process for the preparation of the compounds as claimed in claim 1, which comprises

a) oxidizing a 6-aryltetrahydropyridazinone of the general formula II

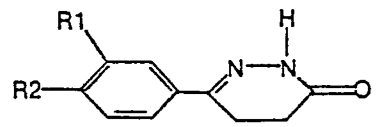
(II).

in which R1 and R2 have the above-mentioned meaning, and, if desired, then converting the resulting pyridazinone into a salt, or

b) reacting a morpholinobutyric acid of the general formula III

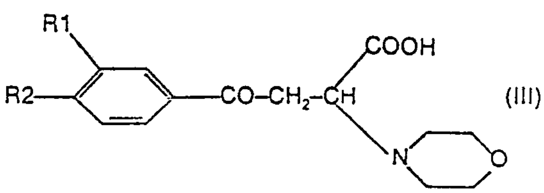
(III)

in which R1 and R2 have the above-mentioned meaning, with hydrazine, and, if desired, then converting the resulting pyridazinone into a salt, or

c) reacting an acrylic acid of the general formula IV,

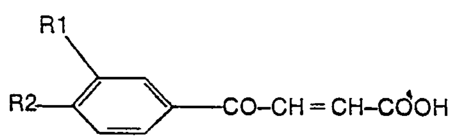
(IV)

in which R1 and R2 have the above-mentioned meaning, and, if desired, then converting the resulting pyridazinone into a salt.

6. Medicaments containing one or more compounds as claimed in claim 1 and/or their pharmacologically tolerated salts.

7. Medicaments as claimed in claim 6 for the treatment of disorders of the bronchi.

8. Medicaments as claimed in claim 7 containing

6-(4-difluoromethoxy-3-methoxyphenyl)-3[2H]pyridazinone

and/or their pharmacologically tolerated salts.

9. Use of the compounds as claimed in claim 1 and/or of their pharmacologically tolerated salts for the preparation of medicaments for the treatment or prophylaxis of diseases based on disorders of the bronchi and/or cardiac insufficiency, or for strengthening the heart.

10. Use of

6-(4-difluoromethoxy-3-methoxyphenyl)-3[2H]pyri-dazinone

and/or of its pharmacologically tolerated salts for the preparation of medicaments for the treatment or prophylaxis of diseases based on disorders of the bronchi and/or cardiac insufficiency, or for strengthening the heart.

**Claims for the contracting state: AT**

1. A process for the preparation of the compounds of the general formula I

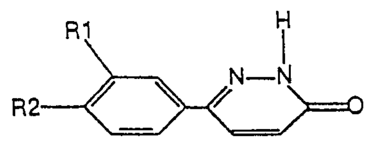
(I).

in which one of the substituents R1 and R2 denotes hydrogen or C1–C4-alkoxy, and the other denotes polyfluoro-C1–C4-alkoxy, and pharmacologically tolerated salts thereof with bases, characterized, in that

a) a 6-aryltetrahydropyridazinone of the general formula II

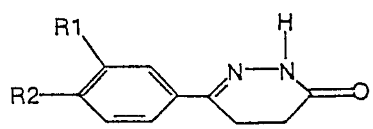
(II)

in which R1 and R2 have the above-mentioned meaning, is oxidized and, if desired, the resulting pyridazinone is then converted into a salt, or

b) a morpholinobutyric acid of the general formula III

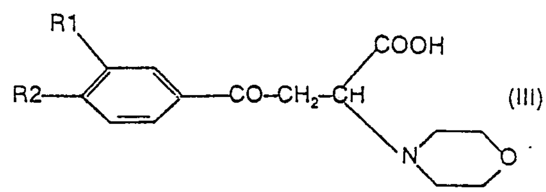
(III)

in which R1 and R2 have the above-mentioned meaning, is reacted with hydrazine and, if desired the resulting pyridazinone is then converted into a salt, or

c) an acrylic acid of the general formula IV

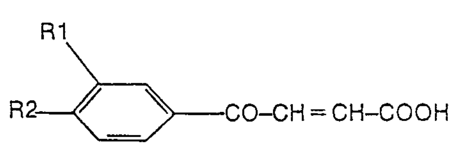
(IV)

in which R1 and R2 have the above-mentioned meaning, is reacted with hydrazine, and, if desired, the resulting pyridazinone is then converted into a salt.

2. A process as claimed in claim 1 for the preparation of compounds of the general formula I, in which one of the substituents R1 and R2 denotes hydrogen or straight-chain C1–C3-alkoxy, and the other denotes polyfluoro-C1–C2-alkoxy, and of their pharmacologically tolerated salts with bases, characterized in that

a) a 6-aryltetrahydropyridazinone of the general formula II, in which R1 and R2 have the above-mentioned meaning, is oxidized and, if desired, the resulting pyridazinone is then converted into a salt, or

b) a morpholinobutyric acid of the general formula III, in which R1 and R2 have the above-mentioned meaning, is reacted with hydrazine, and, if desired, the resulting pyridazinone is then converted into a salt, or

c) an acrylic acid of the general formula IV, in which R1 and R2 have the above-mentioned meaning, is reacted with hydrazine, and, if desired, the resulting pyridazinone is then converted into a salt.

3. A process as claimed in claim 1 for the preparation of compounds of the general formula I, in which one of the substituents R1 and R2 denotes hydrogen or methoxy and the other denotes difluoromethoxy, trifluoromethoxy, 1,1,2,2-tetrafluoroethoxy or 1,1,1-trifluoroethoxy, and pharmacologically tolerated salts thereof with bases, characterized in that

a) a 6-aryltetrahydropyridazinone of the general formula II, in which R1 and R2 have the above-mentioned meaning, is oxidized and, if desired, the resulting pyridazinone is then converted into a salt, or

b) a morpholinobutyric acid of the general formula III, in which R1 and R2 have the above-mentioned meaning, is reacted with hydrazine, and, if desired, the resulting pyridazinone is then converted into a salt, or

c) an acrylic acid of the general formula IV, in which R1 and R2 have the above-mentioned meaning, is reacted with hydrazine, and, if desired, the resulting pyridazinone is then converted into a salt.

4. A process as claimed in claim 1 for the preparation of 6-(4-difluoromethoxy-3-methoxyphenyl)-3[2H]pyridazinone, and pharmacologically tolerated salts thereof with bases, characterized in that

a) 6-(4-difluoromethoxy-3-methoxyphenyl)-tetrahydropyridazin-3-one is oxidized and, if desired, the resulting pyridazinone is then converted into a salt, or

b) 4-(4-difluoromethoxy-3-methoxyphenyl)-2-morpholino-4-oxobutyricacid

is reacted with hydrazine, and the resulting pyridazinone is then converted into a salt, or

c) 2,3-dehydro-4-(4-difluoromethoxy-3-methoxyphenyl)-4-oxobutyric acid is reacted with hydrazine, and, if desired, the resulting pyridazinone is then converted into a salt.

5. A process for the preparation of medicaments for the treatment or prophylaxis of illnesses based on diseases of the bronchi and/or cardiac insufficiency, or for strengthening the heart, characterized in that one or more compounds of the general formula I given in claim 1 and/or pharmacologically tolerated salts thereof with bases, are brought into a form suitable for administration as a medicament by mixing with suitable pharmaceutically acceptable excipients.

6. A process as claimed in claim 5, characterized in that

6-(4-difluoromethoxy-3-methoxyphenyl)-3[2H]pyridazinone

and/or pharmacologically tolerated salts thereof with bases are used.

7. The use of compounds of the general formula I given in claim 1 and pharmacologically tolerated salts thereof with bases in the preparation of medicaments for the treatment or prophylaxis of illnesses based on diseases of the bronchi.

8. The use of 6-(4-difluoromethoxy-3-methoxyphenyl)-3[2H]pyridazinone and pharmacologically tolerated salts thereof with bases in the preparation of medicaments for the treatment or prophylaxis of illnesses based on diseases of the bronchi.

**Revendications pour les Etats contractants: BE, CH, LI, DE, FR, GB, IT, LU, NL, SE**

1. Composés de formule générale I

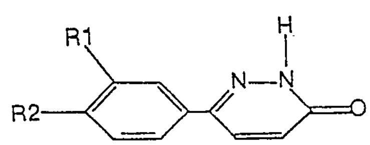

(I),

dans laquelle l'un des substituants R1 et R2 représente un atome d'hydrogène ou un groupe C1–C4-alcoxy et l'autre représente un groupe polyfluoro-C1–C4-alcoxy, et leurs sels, pharmacologiquement compatibles, avec des bases.

2. Composés selon la revendication 1, caractérisé en ce que dans la formule générale I, l'un des substituants R1 et R2 représente un atome d'hydrogène ou un groupe C1–C3-alcoxy linéaire, et l'autre représente un groupe polyfluoro-C1–C2-alcoxy, et leurs sels, pharmacologiquement compatibles, avec des bases.

3. Composés selon la revendication 1, caractérisé en ce que dans la formula générale I, l'un des substituants R1 et R2 représente un atome d'hydrogène ou un groupe méthoxy, et l'autre représente un groupe difluorométhoxy, trifluorométhoxy, 1.1.2.2-tétrafluoroéthoxy ou 1.1.1-trifluoroéthoxy.

4. 6-(4-Difluorométhoxy-3-méthoxyphényl)-3[2H]pyridazinone.

5. Procédé pour la préparation des composés selon la revendication 1, caractérisé en ce que

a) on soumet à une oxydation une 6-aryl-tétrahydropyridazinone de formula générale II

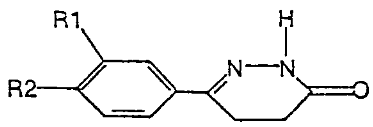

(II),

dans laquelle R1 et R2 sont définis comme spécifié ci-dessus, et, si on le désire, on transforme consécutivement la pyridazinone obtenue en un sel, ou

b) on fait réagir un acide morpholinobutyrique de formule générale III

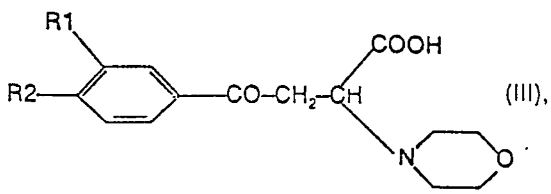

(III),

dans laquelle R1 et R2 sont définis comme spécifié ci-dessus, avec l'hydrazine et, si on le désire, on transforme consécutivement la pyridazinone obtenue en un sel, ou

c) on fait réagir un acide acrylique de formule générale IV

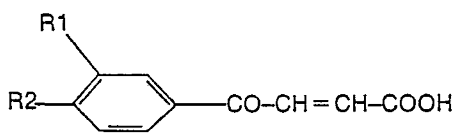

(IV),

dans laquelle R1 et R2 sont définis comme spécifié ci-dessus, avec l'hydrazine et, si on le désire, on transforme consécutivement la pyridazinone obtenue en un sel.

6. Médicament contenant un ou plusieurs composés selon la revendication 1 et/ou leurs sels pharmacologiquement compatibles.

7. Médicament selon la revendication 6 pour le traitement d'affections des bronches.

8. Médicament selon la revendication 7 contenant la 6-(4-difluorométhoxy-3-méthoxyphényl)-3[2H]pyridazinone et/ou ses sels pharmacologiquement compatibles.

9. Application des composés selon la revendication 1 et/ou leurs sels pharmacologiquement compatibles à la préparation de médicaments pour le traitement ou la prophylaxie de maladies, qui découlent d'affections des bronches et/ou d'une insuffisance cardiaque, ou pour tonifier le cœur.

10. Application de la 6-(4-difluorométhoxy-3-méthoxyphényl)-3[2H]pyridazinone ou de ses sels pharmacologiquement compatibles à la préparation de médicaments pour le traitement ou la prophylaxie de maladies, qui découlent d'affections des bronches et/ou d'une insuffisance cardiaque, ou pour tonifier le cœur.

**Revendications pour l'Etat contractant: AT**

1. Procédé pour la préparation de composés de formule générale I

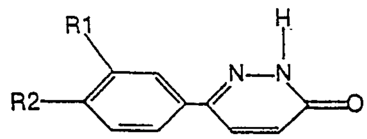

(I),

dans laquelle l'un des substituants R1 et R2 représente un atome d'hydrogène ou un groupe C1–C4-alcoxy et l'autre représente un groupe polyfluoro-C1–C4-alcoxy, et leurs sels, pharmacologiquement compatibles, avec des bases, caractérisé en ce que

a) on soumet à une oxydation une 6-aryl-tétrahydropyridazinone de formula générale II

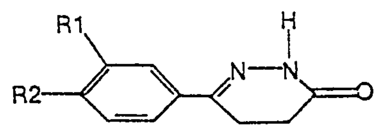

(II),

dans laquelle R1 et R2 sont définis comme spécifié ci-dessus, et, si on le désire, on transforme consécutivement la pyridazinone obtenue en un sel, ou

b) on fait réagir un acide morpholinobutyrique de formula générale III

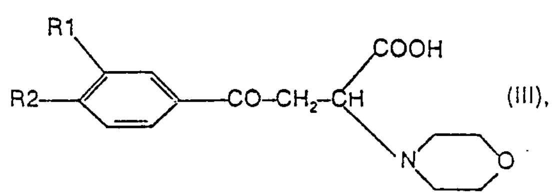

(III),

dans laquelle R1 et R2 sont définis comme spécifié ci-dessus, avec l'hydrazine et, si on le désire, on transforme consécutivement la pyridazinone obtenue en un sel, ou

c) on fait réagir un acide acrylique de formule générale IV

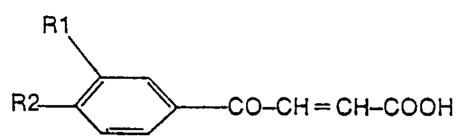

(IV),

dans laquelle R1 et R2 sont définis comme spécifié ci-dessus, avec l'hydrazine et, si on le désire, on transforme consécutivement la pyridazinone obtenue en un sel.

2. Procédé selon la revendication 1 pour la préparation de composés de formule générale I, dans laquelle l'un des substituants R1 et R2 représente un atome d'hydrogène ou un groupe C1–C3-alcoxy linéaire et l'autre représente un groupe polyfluoro-C1–C2-alcoxy, et leurs sels, pharmacologiquement compatibles, avec des bases, caractérisé en ce que

a) on soumet à une oxydation une 6-aryl-tétrahydropyridazinone de formule générale II dans laquelle R1 et R2 sont définis comme spécifié ci-dessus, et, si on le désire, on transforme consécutivement la pyridazinone obtenue en un sel, ou

b) on fait réagir un acide morpholinobutyrique de formule générale III dans laquelle R1 et R2 sont définis comme spécifié ci-dessus, avec l'hydrazine et, si on le désire, on transforme consécutivement la pyridazinone obtenue en un sel, ou

c) on fait réagir un acide acrylique de formule générale IV dans laquelle R1 et R2 sont définis comme spécifié ci-dessus, avec l'hydrazine et, si on le désire, on transforme consécutivement la pyridazinone obtenue en un sel.

3. Procédé selon la revendication 1 pour la préparation de composés de formule générale I, dans laquelle l'un de substituants R1 et R2 représente un atome d'hydrogène ou le groupe méthoxy et l'autre représente un groupe difluorométhoxy, trifluorométhoxy, 1.1.2.2-tétrafluoroéthoxy ou 1.1.1-trifluoroéthoxy, et leurs sels, pharmacologiquement compatibles, avec des bases, caractérisé en ce que

a) on soumet à une oxydation une 6-aryl-tétrahydropyridazinone de formule générale II dans laquelle R1 et R2 sont définis comme spécifié ci-dessus, et, si on le désire, on transforme consécutivement la pyridazinone obtenue en un sel, ou

b) on fait réagir un acide morpholinobutyric de formule générale III dans laquelle R1 et R2 sont définis comme spécifié ci-dessus, avec l'hydrazine et, si on le désire, on transforme consécutivement la pyridazinone obtenue en un sel, ou

c) on fait réagir un acide acrylique de formule générale IV dans laquelle R1 et R2 sont définis comme spécifié ci-dessus, avec l'hydrazine et, si on le désire, on transforme consécutivement la pyridazinone obtenue en un sel.

4. Procédé selon la revendication 1 pour la préparation de la 6-(4-difluorométhoxy-3-méthoxyphényl)-3[2H]pyridazinone, et de ses sels, pharmacologiquement compatibles, avec des bases, caractérisé en ce que

a) on soumet à une oxydation la
6-(4-difluorométhoxy-méthoxyphényl)-tétrahydropyridazin-3-one
et, si on le désire, on transforme consécutivement la pyridazinone obtenue en un sel, ou

b) on fair réagir l'acide
4-(4-difluorométhoxy-3-méthoxyphényl)-2-morpholino-4-oxobutyrique
avec l'hydrazine et consécutivement on transforme la pyridazinone obtenue en un sel, ou

c) on fait réagir l'acide
2,3-déhydro-4-(4-difluorométhoxy-3-méthoxyphényl)-4-oxobutyrique
avec l'hydrazine et, si on le désire, on transforme consécutivement la pyridazinone obtenue en un sel.

5. Procédé pour la préparation de médicaments pour le traitement ou la prophylaxie de maladies, qui découlent d'affections des bronches et/ou d'une insuffisance cardiaque, ou pour tonifier le cœur, caractérisé en ce que l'on amène sous une forme appropriée pour l'administration en tant que médicament, ou en plusieurs composés de formule générale I indiquée dans la revendication 1 et/ou leurs sels, pharmacologiquement compatibles, avec des bases, par mélangeage avec des véhicules pharmaceutiquement acceptables appropriés.

6. Procédé selon la revendication 5, caractérisé en ce qu'on utilise la 6-(4-difluorométhoxy-3-méthoxyphényl)-3[2H]pyridazinone et/ou ses sels, pharmacologiquement compatibles, avec des bases.

7. Application des composés de formule générale I indiquée dans la revendication 1 et de leurs sels, pharmacologiquement compatibles, avec des bases à la préparation de médicaments pour le traitement ou la prophylaxe de maladies qui découlent d'affections des bronches.

8. Application de la 6-(4-difluorométhoxy-3-méthoxyphényl)-3[2H]pyridazinone et de ses sels, pharmacologiquement compatibles, avec des bases à la préparation de médicaments pour le traitement ou la prophylaxe de maladies qui découlent d'affections des bronches.